# DEMANDE DE BREVET EUROPEEN

(11) **EP 2 333 106 A1**
(43) Date de publication de la demande: **15.06.2011**
(21) Numéro de dépôt: 09290922.5
(22) Date de dépôt: 09.12.2009
(51) Int. Cl.: C12Q 1/68

(54) **Utilisation de la diversité combinatoire du répertoire des lymphocytes t comme marqueur pronostic d'un cancer**

(71) Demandeur: ImmunID, 38054 Grenoble (FR)
(72) Inventeur: Manuarii, Manuel, 38600 Fontaine (FR); Caux, Christophe, 01360 Bressolles (FR); Mouret, Jean-Francois, 38500 Coublevie (FR); Menetrier-Caux, Christine, 01360 Bressolles (FR); Pasqual, Nicolas, 38100 Grenoble (FR); Bachelot, Thomas, 69500 Bron (FR); Blay, Jean-Yves, 38290 Frontonas (FR)
(74) Mandataire: Marcadé, Véronique

(57) **Abrégé**

La présente invention concerne une méthode permettant d'identifier, parmi les malades atteints d'un cancer solide donné, ceux qui présentent un risque accru de décès précoce. Cette méthode est basée sur l'analyse *ex vivo* de la diversité lymphocytaire des patients, à partir d'un échantillon biologique contenant des lymphocytes. Une faible diversité lymphocytaire est en effet associée à un mauvais pronostic. Plus particulièrement, il est possible d'établir un seuil de diversité, dépendant de la technologie d'analyse employée et du cancer dont est atteint le malade, en-deçà duquel l'espérance de vie du malade est significativement inférieure à celle, en général, des malades atteints de la même pathologie.

## Description

La présente invention concerne le domaine du pronostic en oncologie. Plus précisément, la présente invention vise à identifier, parmi les malades atteints d'un cancer solide donné, ceux qui présentent un risque accru de décès précoce, afin notamment de pouvoir leur offrir un suivi médical plus approprié, l'accès à des innovations thérapeutiques et d'espérer ainsi augmenter leurs chances de guérison ou au moins leur durée et qualité de vie. Elle vise par ailleurs à identifier un groupe à risque (ne répondant vraisemblablement pas au traitement de référence) pour une pathologie tumorale donnée, afin d'évaluer l'efficacité de nouvelles stratégies thérapeutiques et d'augmenter le rapport bénéfice/risque de candidats médicaments, favorisant ainsi leur approbation clinique.

Les cancers solides désignent la multiplication anormale de cellules dans des tissus et/ou des organes "solides" comme le sein ou la prostate, par opposition à la leucémie, un cancer qui touche le sang et la moelle osseuse.

Parmi les cancers solides, le cancer du sein est un des plus fréquents. Il s'agit essentiellement d'un cancer de la femme (en France, presque 10% des femmes développent un cancer du sein au cours de leur vie). Il est rare chez l'homme (moins d'un cancer du sein sur 100) mais est plus grave, le diagnostic étant souvent plus tardif. C'est le cancer le plus fréquent chez la femme et la première cause de mortalité parmi les cancers gynécologiques des pays développés. Si une partie de ces cancers (10 à 15 %) ont une origine génétique héréditaire, 85 à 90 % des cas ont des origines mal comprises (forme dite sporadique ou non-héréditaire).

Le cancer du sein est une masse résultant de la multiplication de cellules malignes dans la glande mammaire. Cette masse, si elle n'est pas dépistée et traitée, peut grossir et donner des métastases.

Il existe un grand nombre de traitements disponibles pour le cancer du sein, suivant le stade d'évolution et les paramètres inhérents au patient. Chaque situation doit être individualisée et traitée de façon optimale. Pour le cancer du sein localisé, le traitement a un objectif curatif. Il repose sur les cinq armes thérapeutiques que sont la chirurgie, la chimiothérapie, la radiothérapie, l'hormonothérapie et, plus récemment, l'immunothérapie. La chirurgie est l'étape indispensable du traitement curatif du cancer du sein, les autres traitements visant généralement à réduire le risque de métastase ou de rechute. Ils sont donc indiqués si le risque est important et si le bénéfice supposé du traitement est suffisant, car tous ces traitements ont des effets secondaires. Le bénéfice attendu doit donc être mis en balance avec le risque de complication.

Pour le cancer du sein métastatique, il est rare de pouvoir proposer un traitement curatif. Mais les traitements modernes permettent souvent de prolonger la vie du patient de plusieurs années. Le traitement du cancer du sein métastatique repose d'abord sur la chimiothérapie et l'hormonothérapie. Un traitement chirurgical ou par radiothérapie des sites métastatiques peut être envisagé soit dans un but curatif lorsque tous les sites sont accessibles à un traitement (par exemple dans le cas de métastases hépatique ou vertébrale unique) soit dans un but palliatif (par exemple, irradiation d'une métastase osseuse douloureuse).

De nouvelles stratégies thérapeutiques, notamment basées sur l'utilisation d'anticorps monoclonaux ou sur la stimulation du système immunitaire, sont évaluées et développées à un rythme soutenu. Par exemple, le Trastuzumab (Herceptin ®) cible le récepteur Her2 (ou CerbB2), qui est un récepteur membranaire permettant d'activer une des voies de la prolifération cellulaire, sur-exprimé dans 25% des cancers mammaires, souvent de mauvais pronostic. L'Herceptin® a d'abord été utilisée en situation palliative. Dans ce contexte, l'Herceptin® a permis, en moyenne, de doubler le temps de survie de ces patientes. Ajoutée à la chimiothérapie adjuvante, l'Herceptin ® en perfusion tous les 21 jours, pendant 12 mois, réduit de moitié le risque de rechute chez les patientes Her2+ et d'environ un tiers la mortalité.

Malgré ces avancées, la mortalité du cancer du sein reste importante, surtout lorsqu'il atteint un stade métastatique. Il existe toutefois une grande variabilité individuelle, avec une durée de survie allant de moins d'un mois à plusieurs années.

Plusieurs marqueurs de pronostic ont été étudiés pour déterminer si un patient atteint d'un cancer solide donné a un risque accru de décès précoce. A ce titre, on peut citer le taux d'hémoglobine, la présence de métastases hépatiques, le PS *(Performance Status :* quantification de l'état général du patient ; le PS est donc un chiffre représentatif de cet état général) ou le taux de polynucléaires neutrophiles (PNN) (Ray-Coquard et al., 2009) et la lymphopénie, particulièrement la lymphopénie T CD4+ identifiée par les inventeurs de la présente invention (Borg et al., 2004).

La présente invention propose un nouveau marqueur pronostic de décès précoce pour les patients atteints de cancer solide, plus puissant que certains marqueurs déjà décrits, et indépendant de ces derniers. Les inventeurs ont en effet mis en évidence qu'une diminution de la diversité immunitaire des lymphocytes T dans le sang est corrélée à un risque accru de décès précoce.

Chaque lymphocyte T fonctionnel possède un récepteur (TCR) qui reconnaît de façon spécifique un nombre limité de peptides antigéniques différents. De ce fait, un vaste répertoire de récepteurs est requis pour assurer la défense de l'individu contre les multiples infections, les proliférations malignes ou autres agressions qu'il est susceptible de rencontrer dans son environnement. Pour cela, le système immunitaire a développé un mécanisme d'assemblage d'un grand nombre de segments de gènes V, D, J positionnés de façon discontinue dans le génome. Ce mécanisme d'assemblage, appelé "recombinaison V(D)J", est indépendant d'une cellule à l'autre et permet d'obtenir un seul "fragment" de gène codant pour le TCR. Ce système permet, avec un nombre modeste de gènes, de générer un grand nombre de récepteurs différents. Chaque cellule utilise une combinaison de segments géniques selon des règles précises et obtient une chaîne TCR potentiellement unique.

Le principe de la recombinaison est basé sur la reconnaissance de séquences RSS spécifiques des gènes V(D)J et l'excision de la région chromosomique intercalée entre les deux gènes réarrangés. Chaque gène V et J possède, à une de ses extrémités, une séquence signal de recombinaison (RSS). Quant aux gènes D, ils en possèdent aux deux extrémités. Les RSS sont des séquences reconnues par les enzymes spécifiques de la recombinase, RAG I et RAG II, exprimées spécifiquement dans les lymphocytes. Ces protéines sont les actrices principales du réarrangement. Une fois associées aux protéines HMG (*High mobility group*)*,* les enzymes RAG reconnaissent le nonamère du RSS grâce à leur homéodomaine et induisent une coupure entre le segment de gène V, D, J et l'heptamère, de façon à générer une extrémité codante et une extrémité signal. L'achèvement d'un réarrangement est obtenu après ligation des deux extrémités codantes V et J. Cette étape est précédée par l'action de l'enzyme TdT et d'une nucléase au niveau de la jonction V-J. Une fois réarrangé, le gène néoformé est transcrit puis épissé en ARNm avant d'être traduit en protéine membranaire.

Quatre mécanismes majeurs contribuent à générer la diversité du répertoire : 1) une diversité combinatoire qui correspond à la première étape de réarrangement entre un segment V et un segment J, éventuellement séparés par un segment D ; 2) une diversité jonctionnelle, générée au niveau de la jonction entre les segments de gènes réarrangés ; 3) des hypermutations somatiques dans les gènes réarrangés V-J et V-D-J ; 4) une diversité d'appariement des hétérodimères protéiques TCRα x TCRβ ou TCRγ x TCRδ.

La première étape de génération de diversité, appelée ici "diversité combinatoire", est basée sur le principe du réarrangement des gènes V(D)J. Le calcul de cette diversité consiste à estimer le nombre de combinaisons mV x nD x pJ possibles. Cette première étape de génération de diversité définit l'ordre de grandeur du répertoire. En effet, même si cette étape ne génère qu'une modeste variabilité de combinaison (de l'ordre de quelques milliers de combinaisons possibles par rapport au répertoire maximal théorique estimé à 10¹⁵ (Davis and Bjorkman, 1988), la diversité combinatoire maximale est directement liée au nombre de gènes V, D et J initialement disponibles : les deux autres étapes de génération de la diversité amplifient de manière exponentielle la diversité du répertoire primaire.

La diversité de jonction permet de générer une très grande variabilité au niveau de la région CDR3 du récepteur en contact avec le peptide antigénique. Deux mécanismes contribuent à augmenter la diversité jonctionnelle : 1) le premier mécanisme est dû à l'ajout de nucléotides P (pour palindromiques), provenant de la résolution de l'épingle à cheveux des segments réarrangés (Fugmann et al., 2000). La diversité générée n'est pas aussi grande que celle provenant du deuxième mécanisme faisant intervenir l'enzyme terminal deoxynucleotidyl transférase ; 2) la TdT produit une importante diversité, en ajoutant de façon aléatoire des nuclétotides N à l'extrémité 3' de chaque segment codant, sans besoin de matrice génomique (Bogue et al., 1992).

Le mécanisme des réarrangements secondaires contribue à "conserver" de la diversité : la diversité jonctionnelle représente le plus grand facteur d'amplification de la diversité du répertoire, mais s'il n'y avait pas le mécanisme de réarrangement secondaire pour sauver 2/3 des thymocytes ayant interrompu leur cadre de lecture, ce bénéfice en terme de diversité représenterait un coût important pour l'organisme, avant même l'étape de sélection positive. Ces réarrangements non productifs ne peuvent donner une protéine TCR fonctionnelle. La cellule a alors la possibilité de tenter un deuxième réarrangement avec les gènes V(D)J encore disponibles sur le locus. La propriété d'ouverture concentrique du locus TRAD favorise ce processus en laissant le plus de chances possibles à la cellule, puisque les premiers réarrangements effectués par la cellule ont lieu entre un couple de gène V-J proches l'un de l'autre (Pasqual et al., 2002). Si ces premiers réarrangements ne sont pas productifs, la cellule a la possibilité de tenter des réarrangements sur son second chromosome, ou encore d'utiliser les gènes V et J disponibles de part et d'autre du premier réarrangement. Ainsi, les réarrangements secondaires permettent la survie d'un grand nombre de cellules qui, à l'issue d'un premier réarrangement non productif, auraient dû être éliminées.

Les hypermutations somatiques (HMS) ont lieu pendant la différenciation des lymphocytes B dans les ganglions lymphatiques, lors de la rencontre avec un antigène. Les HMS sont situées dans des *"hot spot motifs"* des gènes réarrangés V-J et V(D)J des Ig (Chaudhuri et al., 2003) mais aussi dans certains cas, dans des gènes réarrangés V-J et V(D)J des TCR (Kotani et al., 2005). Le TCR peut être la cible d'HMS au niveau des gènes variables, si le lymphocyte surexprime l'enzyme AID (*Activation-induced cytidine deaminase*) qui est normalement spécifique des lymphocytes B. En temps normal le TCR ne subit pas d'HMS car le lymphocyte T ne synthétise tout simplement pas l'AID. Néanmoins, si le lymphocyte T se met à l'exprimer, le TCR est aussi sensible à cette enzyme que le BCR car il possède toutes les séquences sur laquelle elle agit. Au total, il est décrit dans la littérature que ce mécanisme induit une diversité supplémentaire d'un facteur 1000 dans le but d'augmenter les chances de reconnaître un antigène.

L'estimation de la diversité issue de l'appariement entre une chaîne TCRα et une chaîne TCRβ est obtenue en multipliant le nombre de combinaisons différentes d'une chaîne TCRα, par le nombre de combinaisons possibles pour la chaîne TCRβ. La diversité générée par ce mécanisme est directement dépendante du nombre de combinaisons primaires obtenu lors du réarrangement. En effet, si on examine le nombre de combinaisons primaires TCRγδ chez la souris, sans prendre en compte la diversité jonctionnelle, le résultat est seulement de 40 TCRδ (=10V*2D*2J) x 28 TCRγ (=7V*4J) = 1120 combinaisons différentes, alors que le même calcul conduit à 5,6 106 combinaisons pour TCRαβ (calculé comme suit : 102Vα*60Jα*33Vβ*2Dβ*14Jβ).

La diversité du répertoire des immunoglobulines produites par les lymphocytes B résulte des mêmes mécanismes que ceux décrits ci-dessus pour les lymphocytes T.

La mesure de la diversité immunologique permet entre autres d'étudier les mécanismes de mise en place du répertoire immunitaire, l'homéostasie, les lymphocytes T ou B impliqués dans une réponse immunitaire, dans une leucémie ou encore d'évaluer l'immunodéficience induite par un traitement ou une pathologie, en particulier tumorale, ou à l'inverse l'activation du système immunitaire spécifique. Cette liste n'est pas exhaustive.

L'étude du répertoire immunitaire d'une population lymphocytaire a conduit au développement de plusieurs approches multiparamétriques, permettant à la fois de mesurer le niveau de diversité et d'identifier la présence de certains clones T ou B spécifiques. Certaines approches mises au point par les immunologistes pour évaluer ces différents niveaux de diversité sont listées ci-dessous selon le principe et le "niveau" de diversité mesurée.

### Mesure de la diversité V

- Par cytométrie (Van den Beemd, van Dongen et al. 2000)
- Par Q-PCR au niveau génomique et transcriptomique (Fuschiotti et al., 2007; Pasqual et al., 2002).
- Par séquençage

### Mesure de la diversité jonctionnelle CDR3 :

- Par Immunoscope® (Cochet et al., 1992; Pannetier et al., 1995).
- Q-PCR couplé à l'immunoscope (TcLandscape®)
- Par séquençage
- Par la méthode Amplicot au niveau génomique (Baum and McCune, 2006).
- Par puce à ADN (Bonarius et al., 2006).

### Etude des hypermutations somatiques (HMS) :

- PCR / séquençage (Hamblin et al., 1999).

### Mesure indirecte via la décroissance des cercles d'excisions TREC's

- Par PCR (Douek et al., 1998).
- Par Q-PCR (Pham et al., 2003).

Si certaines de ces approches ont déjà fait leurs preuves en recherche fondamentale, notamment l'Immunoscope® (Pannetier, C., J. Even, et al., 1995) ou la cytométrie en flux (Van den Beemd, van Dongen et al., 2000), il reste encore un certain nombre de validations scientifiques et techniques à apporter pour évaluer la pertinence de leur utilisation comme bio-marqueur médical. Face à la complexité du système immunitaire, le scientifique aurait besoin de coupler des approches technologiques complémentaires pour décrypter l'ensemble des informations contenues dans le répertoire immunitaire et relevantes d'une pathologie donnée.

D'autres méthodes, basées sur l'utilisation de PCR amplifiant spécifiquement des segments d'acides nucléiques caractéristiques de certains réarrangements, ont été décrites. Par exemple, les brevets US 5,296,351 et US 5,418,134 présentent une méthode de détection des leucémies lymphoïdes ou des lymphomes B ou T, basée sur l'amplifications de séquences codant pour des immunoglobulines et/ou des récepteurs T, utilisant des amorces "consensus" pour amplifier simultanément plusieurs réarrangements V-J.

Les inventeurs ont précédemment décrit des méthodes et kits pour mesurer la diversité combinatoire du répertoire des lymphocytes T et/ou B d'un individu (WO 2009/095567). Dans cette demande de brevet, les inventeurs ont également défini la "divpénie" comme un déficit de diversité combinatoire immunitaire, et évoqué le risque de mortalité par infection accru pour des patients en état de divpénie.

Dans le présent texte, la divpénie désigne un déficit de diversité immunitaire, quel que soit le niveau (diversité combinatoire, jonctionnelle ou autre) auquel cette diversité est mesurée. La divpénie T désigne donc un déficit de diversité du répertoire des lymphocytes T.

Dans les études exposées dans la partie expérimentale ci-dessous, les inventeurs ont cherché à déterminer s'il existait une relation entre la divpénie et différents risques à prendre en considération lors du traitement de malades atteints de cancers solides, notamment au stade métastatique. De façon surprenante, ils ont constaté que la divpénie T avait une corrélation directe, forte, et indépendante des autres facteurs de risques connus, avec la mortalité précoce (pas nécessairement par infection), en particulier dans le cas du cancer du sein métastatique (exemple 1). En particulier, ils ont déterminé, dans le cas de ce cancer, qu'une diversité combinatoire des réarrangements V(D)J des gènes du locus hTRB inférieure à 20% des réarrangements possibles est un facteur pronostic puissant de décès précoce, avec une médiane de survie inférieure à 6 mois. Ceci ne semble pas être le cas de la divpénie B (exemple 2). En outre, contrairement à ce qui aurait pu être attendu, la divpénie (T ou B) ne semble pas être un facteur de risque de toxicité grave des traitements chimiothérapeutiques utilisés pour le traitement du cancer du sein primaire (résultats non montrés).

L'identification rapide, et avant tout traitement, de patients ayant un risque accru de mortalité précoce a des conséquences importantes pour ces patients et pour la recherche médicale, car elle permet d'envisager un suivi particulier pour ces malades, le cas échéant avec une hospitalisation plus soutenue et/ou l'administration de traitements moins immunosuppresseurs ou plus ciblés sur la stimulation de leur système immunitaire. Pour la recherche médicale, elle permet d'identifier une population de patients homogène pour laquelle le traitement de référence sera vraisemblablement inefficace et pour laquelle les cliniciens semblent actuellement particulièrement démunis. La caractérisation d'une telle population représente un enjeu majeur pour les cliniciens et les industries pharmaceutiques pour les essais cliniques de traitements innovants qui auront une efficacité supérieure à celle des traitements de référence, augmentant ainsi la possibilité d'enregistrement de nouveaux médicaments. Cette population pourra être de préférence sélectionnée pour effectuer des essais cliniques pour tester des traitements innovants.

La présente invention porte donc en premier lieu sur l'utilisation de la diversité du répertoire des lymphocytes T d'un individu atteint d'un cancer solide, comme marqueur pronostique de l'évolution de ce cancer. En particulier, en cas de divpénie T, ce marqueur est indicatif d'un risque accru de décès précoce.

Plus particulièrement, la présente invention concerne une méthode permettant de déterminer *ex vivo* ou *in vitro* si un patient atteint d'un cancer solide a un risque accru de décès précoce, autrement dit pour établir *ex vivo* ou *in vitro* un pronostic pour un individu souffrant d'un cancer solide, comprenant les étapes suivantes :
(i) à partir d'acide nucléique (par exemple, d'ADN génomique ou d'ARN messager) provenant d'un échantillon biologique contenant des lymphocytes dudit individu, mesurer le niveau de diversité du répertoire des lymphocytes T dudit individu ;
(ii) comparer le niveau de diversité mesuré à l'étape (i) à un seuil prédéterminé ;
(iii) en déduire, dans le cas où le niveau de diversité mesuré à l'étape (i) est inférieur au seuil prédéterminé, que l'individu a un risque élevé de décès précoce.

Un exemple particulier de cancer solide pour lequel cette méthode est appropriée est le cancer du sein, mais en se basant sur les travaux précédents des inventeurs sur la lymphopénie (Borg et al., 2004; Ray-Coquard et al., 2009) elle peut également être transposée à tous les cancers solides, tels que les cancers de la prostate, du poumon, du côlon, ovariens, de la sphère ORL, les lymphomes, les sarcomes, *etc.*

La méthode de l'invention est particulièrement appropriée pour établir un pronostic pour un patient atteint d'un cancer solide métastatique.

Bien évidemment, le seuil considéré à l'étape (ii) peut dépendre du profil clinique du patient (type et stade de cancer, et le cas échéant âge et autres paramètres physiologiques), et l'homme du métier est à même de conduire les expérimentations nécessaires, par des études rétrospectives ou prospectives, pour définir un seuil pertinent pour un type de pathologie et/ou de patient donné.

De même, la notion de "mortalité précoce" est à rapporter au type de cancer dont est atteint l'individu, ainsi qu'au stade d'avancement de ce cancer (notamment, savoir s'il est métastatique ou non). On parlera ici de "risque accru de mortalité précoce" pour un patient lorsqu'il appartient, dans une cohorte représentative de son état pathologique, à une sous-population dont la médiane de survie est inférieure à celle de la cohorte entière (un exemple de cohorte représentative de patients atteints de cancer du sein métastatique est donné dans la partie expérimentale). Pour une sous-population donnée (par exemple, patients en état de divpénie T), on parlera de mortalité précoce lorsque la médiane de survie de cette sous-population (représentative de l'espérance de vie des membres de cette sous-population) est statistiquement significativement inférieure à celle de la cohorte entière. Dans le cadre de la présente invention, la mortalité précoce des malades en état de divpénie T correspond typiquement à une espérance de vie deux ou trois fois moindre, voire 5 fois moindre que celle d'une population de malades atteints du même cancer au même stade, sans qu'il soit tenu compte de leur niveau de diversité immunitaire.

Un autre paramètre susceptible de jouer sur le seuil de diversité des lymphocytes T en dessous duquel la divpénie T constitue un marqueur significatif de risque de mortalité précoce est la technologie avec laquelle est mesurée cette diversité. En effet, comme mentionné plus haut, l'homme du métier dispose d'un grand nombre de technologies (séquençage, immunoscope, puce à ADN, ...) pour mesurer, à différents niveaux, la diversité du répertoire des lymphocytes T d'un individu. La présente invention peut être mise en oeuvre en utilisant n'importe quelle technologie mesurant la diversité des lymphocytes T, quel que soit le matériel d'origine (ADNg, ARN, etc...) et le niveau auquel est mesurée cette diversité (diversité combinatoire, jonctionnelle, etc.). La transposition des résultats décrits dans la partie expérimentale ci-dessous, par des expérimentations et une analyse statistique de routine, permettra de déterminer un seuil significatif pour la technologie alternative utilisée.

Selon un mode de réalisation particulier de l'invention, illustré expérimentalement ci-dessous, la diversité est mesurée au niveau combinatoire. Les inventeurs, dans la demande de brevet publiée sous le numéro WO 2009/095567, ont décrit plusieurs méthodes de mesure de la diversité combinatoire, selon le(s) locus ciblé(s) (TRA, TRB, TRG, TRD), l'analyse ou non des réarrangements incomplets, le pourcentage, pour chaque locus, de réarrangements analysés (en effectuant un nombre plus ou moins important de PCR), le niveau d'analyse de ces réarrangements (détection du pourcentage de réarrangements ou quantification de chaque réarrangement observé et précisément identifié), *etc.*

Dans le cadre de l'étude expérimentale exposée ci-dessous, la diversité combinatoire du répertoire des lymphocytes T a été évaluée à partir de l'analyse des réarrangements V(D)J du seul locus TRB, par une méthode permettant d'analyser plus de 80% des réarrangements V(D)J du locus TRB. Une méthode dans laquelle la technologie utilisée à l'étape (i) permet d'analyser au moins 70% des réarrangements V(D)J du locus TRB constitue d'ailleurs un mode de mise en oeuvre préféré de l'invention. En particulier, la méthode de l'invention peut être avantageusement mise en oeuvre en effectuant la mesure de la diversité combinatoire du répertoire des lymphocytes T par des PCR multi-n-plexes avec n≥2 à l'aide de combinaisons d'au moins 3 amorces, chaque combinaison d'amorces comprenant au moins les amorces hTRBJ1.6 (CTTGGTGCATGGCTATGTAATCCTG, SEQ ID No : 1), hTRBJ2.7 (CTCGCCCTCTGCTCAGCTTTCC, SEQ ID No : 2) et une amorce hTRBV choisie dans le groupe constitué des amorces de SEQ ID Nos : 3 à 25. Une méthode selon l'invention, dans laquelle l'analyse du locus TRB est réalisée en effectuant au moins 23 PCR multi-2-plexes, chaque PCR multi-2-plexe étant réalisée avec un triplet d'amorces constitué des amorces hTRBJ1.6 (SEQ ID No : 1), hTRBJ2.7 (SEQ ID No : 2) et d'une amorce hTRBV choisie dans le groupe constitué des amorces de SEQ ID Nos : 3 à 25 (Tableau 1), constitue un mode de mise en oeuvre préféré de l'invention.

**Tableau 1 : amorces s'hybridant au locus TRB (gènes V), utilisables dans le cadre de la présente invention**

| Nom du gène | Nom de l'amorce | Taille (nt) | Distance entre l'extrémité 5' de l'amorce et la fin du gène V (pb) | Séquence | SEQ ID N° |
|---|---|---|---|---|---|
| TRBV2 | hTRBV2up2 | 26 | 255 | CACACAGATGGGAC AGGAAGTGATCT | 3 |
| TRBV4 | hTRBV4up_ex | 23 | 100 | GCTTCTCACCTGAAT GCCCCAAC | 4 |
| TRBV5.1, 3, 4, 5, 6, 8 | hTRBV5up_ex1/2 | 25 | 256 | CTGATCAAAACGAG AGGACAGCAAG | 5 |
| TRBV5.7 | hTRBV5up_ex2/2 | 25 | 256 | CTGATCAAAACGAG AGGACAGCACG | 6 |
| TRBV6.4 | hTRBV6up_ex2/2 | 23 | 279 | GATCACCCAGGCAC CAACATCTC | 7 |
| TRBV7.2 | hTRBV7up_ex2/3 | 25 | 301 | CAGATCACACAGGA GCTGGAGTCTC | 8 |
| TRBV7.9 | hTRBV7up_ex3/3 | 27 | 303 | CACAGATCACGCAG ATACTGGAGTCTC | 9 |
| TRBV9 | hTRBV9up_ex | 23 | 92 | CGCACAACAGTTCCC TGACTTGC | 10 |
| TRBV11 | hTRBV11up_ex | 27 | 120 | TTCACAGTTGCCTAA GGATCGATTTTC | 11 |
| TRBV12.1 | hTRBV12.1up1 | 27 | 196 | TTCTCTGGTACAGAC AGACCTTTGTGC | 12 |
| TRB V12.2 | hTRBV12.2up 1 | 27 | 196 | TTTTCTGGTACAGAG ATACCTTCGTGC | 13 |
| TRBV13 | hTRBV13up1 | 25 | 356 | GTTGCTGAAGTGTCA AACTCTCCCG | 14 |
| TRBV14 | hTRBV14up_ex | 24 | 271 | TCCCCAGCCACAGC GTAATAGAGA | 15 |
| TRBV15 | hTRBV15up_ex | 24 | 163 | CCCCAAAGCTGCTGT TCCACTACT | 16 |
| TRBV16 | hTRBV16up1 | 22 | 295 | CTCCTGGTGAAGAA GTCGCCCA | 17 |
| TRBV18 | hTRBV18up1 | 22 | 46 | TAGTGCGAGGAGAT TCGGCAGC | 18 |
| TRBV19 | hTRBV19up2 | 24 | 217 | CTGGGAGCAAGTGA GTCCTGGGT | 19 |
| TRBV20 | hTRBV20-1up_ex | 24 | 91 | TCATCAACCATGCAA GCCTGACCT | 20 |
| TRBV24 | hTRBV24up_ex | 24 | 96 | AGTGTCTCTCGACAG GCACAGGCT | 21 |
| TRBV25 | hTRBV25up_int | 23 | 273 | CCTCTTTGTTGGGTT TGTGCCTG | 22 |
| TRBV27 | hTRBV27up2 | 22 | 312 | GTCCCCTTCCTTTAC AGGCCCC | 23 |
| TRBV29 | hTRBV29up_G | 21 | 91 | CCATCAGCCGCCCA AACCTAA | 24 |
| TRBV30 | hTRBV30up1 | 26 | 148 | TGCTCTTCTACTCCG TTGGTATTGGC | 25 |

Le cas échéant, l'homme du métier peut choisir d'évaluer la diversité combinatoire du répertoire des lymphocytes T en examinant un nombre plus limité de réarrangements V(D)J du locus TRB, et/ou en examinant des réarrangements d'un autre locus choisi parmi les locus TRA, TRG et TRD. Le pourcentage de diversité du répertoire T du patient sera alors extrapolé, en calculant le pourcentage de réarrangements observés parmi les réarrangements théoriquement observables avec la technologie utilisée. Bien entendu, le résultat obtenu sera d'autant plus informatif que la technologie utilisée permettra l'observation théorique d'un nombre élevé de réarrangements. Le coût de l'analyse sera donc mis en balance avec le niveau d'information souhaité, selon les situations, pour déterminer un nombre optimum de réarrangements observables. En tout état de cause, il est préférable, pour obtenir un résultat exploitable, d'analyser au moins 10 réarrangements, de préférence au moins 20, ou 30, voire 50 réarrangements du locus TRA ou du locus TRB. De manière encore préférée, on utilisera une technologie qui permet d'observer au moins 20% des réarrangements théoriques possibles d'un de ces locus (299 réarrangements V-J pour le locus TRB et 2500 pour le locus TRA).

Pour la mise en oeuvre de l'invention, l'ADN génomique est de préférence purifié. Toutefois, l'homme du métier peut, en fonction de l'évolution des technologies, choisir de travailler sur des échantillons bruts. Tout échantillon biologique susceptible de contenir des lymphocytes T peut être utilisé ; à titre d'exemples non limitatifs d'échantillons utilisables, on peut citer des échantillons de sang (sang total ou PBMC par exemple), thymus, ganglion, rate, sein, foie, peau, ou plus généralement tout échantillon tumoral, ainsi qu'un fluide biologique tel qu'un épanchement pleural ou un ascite.

Selon une mise en oeuvre préférée de l'invention, le seuil de diversité immunitaire auquel sera comparée la diversité d'un patient atteint de cancer métastatique sera prédéterminé de façon à ce que l'espérance de survie d'un patient dont le niveau de diversité est inférieur à ce seuil est au moins deux fois moindre que l'espérance de survie généralement observée pour les patients atteints du même cancer solide métastatique que celui dont il est atteint.

Selon une mise en oeuvre particulière de l'invention, illustrée dans l'exemple 1 sur une cohorte de patientes atteintes de cancer du sein métastatique, le seuil considéré à l'étape (ii) du procédé est fixé à 30% de diversité, et l'interprétation à l'étape (iii) consiste à dire que si le niveau de diversité combinatoire des lymphocytes T mesuré est inférieur à ce seuil, l'individu a une espérance de vie deux fois moindre que celle généralement observée pour sa pathologie. Bien évidemment, les réserves exposées plus haut restent pertinentes, et l'homme du métier pourra, sur un type ou un stade différent de cancer, et/ou en utilisant une technologie différente de l'analyse de la diversité combinatoire des réarrangements du locus TRB, ajuster le seuil, de façon à obtenir un seuil de diversité en deçà duquel l'espérance de vie des patients est deux fois moindre que pour les patients atteints de la même pathologie, indépendamment de leur statut immunologique.

Selon une autre mise en oeuvre particulière de l'invention, également illustrée à l'exemple 1, le seuil considéré à l'étape (ii) du procédé est fixé 20% de diversité, et l'interprétation à l'étape (iii) consiste à dire que si le niveau de diversité combinatoire des lymphocytes T mesuré est inférieur à ce seuil, l'individu a une espérance de vie cinq fois moindre que celle généralement observée pour sa pathologie. L'invention porte plus spécifiquement sur une méthode telle que décrite ci-dessus, dans laquelle le patient est atteint d'un cancer du sein métastatique, et dans laquelle un niveau de diversité (en particulier, de diversité combinatoire) des réarrangements V(D)J du locus TRB inférieur à 20% est indicatif d'une espérance de survie du patient inférieure à 6 mois (p= 2.10⁻⁷).

Il est particulièrement intéressant de noter que le marqueur objet de la présente invention est indépendant des autres facteurs de risque identifiés à ce jour (p= 0.0092). En conséquence, la méthode selon l'invention peut être mise en oeuvre pour établir un pronostic sans prendre en compte ces marqueurs, en particulier sans prendre en compte la numération lymphocytaire du patient. La divpénie T peut toutefois, en accord avec l'invention, être combinée avec d'autres paramètres, immunitaires tels que le niveau de cytokines sériques, en particulier de type IL7 ou IL15, ou le nombre de cellules CD4+, ou encore avec d'autres paramètres biologiques ou cliniques tels que l'âge, le *performance status,* la numération lymphocytaire, le nombre de cellules CD4+ ou le niveau d'hémoglobine, pour établir un pronostic chez un patient atteint d'un cancer solide.

Un autre aspect de la présente invention est de sélectionner des patients à risque pour les inclure dans des protocoles cliniques innovants ayant pour objectifs soit de corriger la lymphopénie et/ou la divpénie (cytokines type IL2, IL7, IL15, immunostimulation, compléments alimentaires,...) soit d'évaluer des thérapies émergentes. La possibilité d'identifier les patients à risque avant tout traitement de phase métastatique notamment, est également susceptible d'augmenter fortement les chances de bénéfice du traitement par rapport au risque, et donc de valider une thérapie innovante par rapport à la thérapie de référence. Dans certains cas, cette approche de stratification des patients permet en parallèle de réduire le coût de l'étude clinique en ne traitant que les patients à risque (un exemple de comparaison des coûts est donné à l'exemple 3 ci-dessous, à titre purement indicatif).

L'invention porte donc sur une méthode pour déterminer *ex vivo* ou *in vitro* si un patient atteint d'un cancer solide doit être inclus dans un protocole de recherche clinique pour tester un nouveau médicament, comprenant les étapes suivantes :
(i) déterminer, en mettant en oeuvre une méthode de pronostic telle que décrite plus haut, si le patient a un risque accru de décès précoce, et
(ii) si le patient a un risque accru de décès précoce, l'inclure dans le protocole de recherche clinique.

L'identification, par une méthode telle que décrite ci-dessus, de patients particulièrement à risque, qui ne répondront vraisemblablement pas bien à une thérapeutique conventionnelle ou de référence, permet en outre d'adapter le traitement de ces patients, par exemple pour leur proposer un suivi hospitalier particulier, une antibiothérapie préventive, une immunostimulation médicamenteuse ou par des compléments alimentaires, une vaccination thérapeutique, une chimiothérapie adaptée, de préférence la moins immunosuppressive possible, ou encore un changement de posologie ou de fréquence d'administration d'une chimiothérapie qu'ils reçoivent déjà.

Outre les dispositions qui précèdent, l'invention comprend encore d'autres dispositions, qui ressortiront des exemples expérimentaux ci-dessous, et des figures annexées.

### Légende des figures

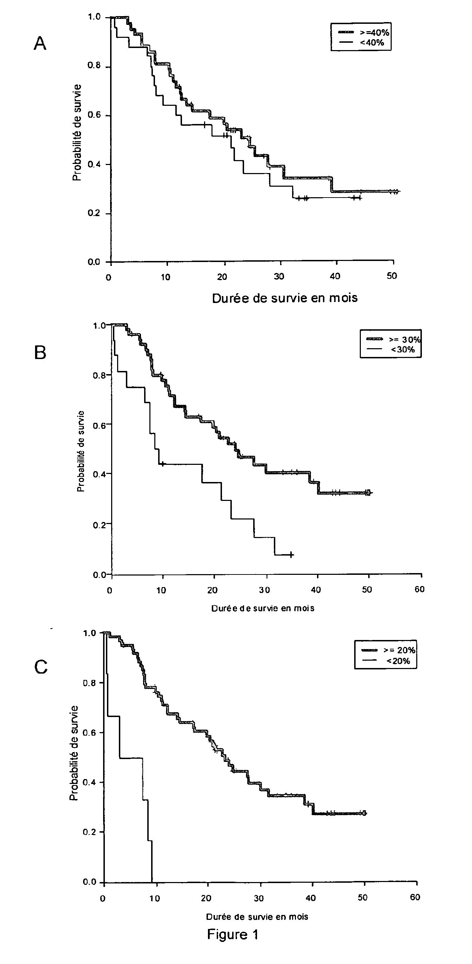

**Figure 1** : Courbes de survie des patientes selon leur diversité hTRVJ. Survie globale avec un seuil de diversité combinatoire TCR de **A**) 40% (en gris épais patientes avec diversité TCR> 40% et en noir diversité TCR ≤40%) (p=0.481). **B**) 30% (en gris épais patientes avec diversité TCR> 30% et en noir diversité TCR ≤30%) (p=0.0047). **C**) 20% (en gris épais patientes avec diversité TCR> 20% et en noir diversité TCR ≤20%) (p=2.10⁻⁷).

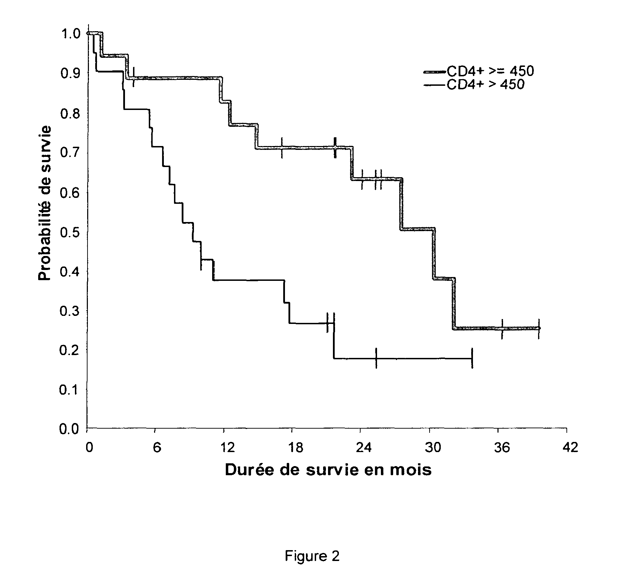

**Figure 2** : Survie globale en fonction de la quantité de lymphocytes T CD4+ circulants (en gris épais patientes avec CD4+>450/µl et en noir CD4+≤450/µl) (p=0.011).

### EXEMPLES

### Exemple 1 : Etude de la corrélation entre un risque accru de décès précoce et une faible diversité combinatoire T, chez des patientes atteintes de cancer du sein métastatique

### Matériels et méthodes

### Sélection des patientes

Patientes incluses dans le protocole clinique SEMTOF mené au CLB : patientes atteintes d'un cancer du sein phase métastatique en 1 ère ligne de chimiothérapie dont le PS (*Performance Status*) est inférieur ou égal à 2. Les patientes inclues ont reçu les traitements de chimiothérapie classiquement utilisés dans cette pathologie. Le prélèvement sanguin sur lequel l'analyse du répertoire immunitaire sera effectuée, est réalisé avant l'administration de la première ligne de chimiothérapie.

### Mesure de la diversité combinatoire T

Une PCR Multiplexe ImmunTraCkeR®β est réalisée en utilisant un oligonucléotide *"upstream"* spécifique de tous les membres d'une famille V donnée et un oligonucléotide *"downstream"* spécifique d'une famille J donnée. Cette technologie permet la détection simultanée de plusieurs réarrangements V-J dans la même réaction. Le test ImmunTraCkeR®β est composé de 23 puits (+1 puits pour le contrôle qualité interne), chacun capable de détecter l'ensemble des réarrangements d'une famille V. Ce test permet de détecter 276 réarrangements hTRB V-J différents (276 réarrangements observés correspondraient donc ici à 100% des réarrangements observables). De la même manière, il est possible de détecter les 48 réarrangements hIgH V-J possible, assurant une couverture complète de ce répertoire. Les conditions de PCR ont été décrites précédemment (Marodon et al., 2009).

La description de l'estimation semi-quantitative et qualitative du répertoire est décrite ci-dessous.

Attribution des réarrangements : cette attribution consiste à rapprocher la taille des produits de PCR mesurée avec un standard dont la taille et la concentration de chaque produit sont connues. Cette analyse s'effectue soit de façon manuelle soit grâce au logiciel Constel'ID développé par ImmunID.

Evaluation semi quantitative : La PCR est arrêtée en fin de phase exponentielle d'amplification. Le signal est mesuré en fonction de l'intensité de fluorescence de notre marqueur par une caméra CCD du type puce à ADN. La quantification du signal numérique est assurée par un logiciel d'acquisition qui mesure l'intensité de fluorescence des réarrangements V-J détectés, normalisée par rapport au standard de migration.

Evaluation qualitative du répertoire immunitaire : un score de diversité du répertoire est calculé en fonction du nombre maximum de réarrangements attendus (nombre de réarrangements V(D)J présents dans l'échantillon, divisé par le nombre de réarrangements théoriquement observables avec le kit ImmunTraCkeR (en l'occurrence, 276), multiplié par 100). Ces données permettent d'évaluer l'aspect qualitatif du répertoire immunitaire et d'estimer le niveau de perturbation du répertoire suite à un traitement.

### Analyses statistiques

Afin de déterminer le rôle des différents biomarqueurs du cancer dans la survie des patientes, un modèle d'estimation de la survie globale a été construit. Le type de modèle statistique utilisé pour ce faire est le modèle de Cox. La construction du modèle est réalisée sur la base de la mise en présence de différents facteurs pronostiques déjà identifiés pouvant être protecteurs ou à risque de décès du cancer (ex : une faible concentration d'hémoglobine est à risque pour la patiente). La valeur prédictive de chacun de ces différents facteurs est préalablement évaluée individuellement (analyse univariée), afin de déterminer la pertinence de leur intégration dans un modèle multifactoriel (analyse multivariée) de prédiction de la survie globale. Cette évaluation préalable est aussi réalisée par l'application d'un modèle de Cox.

### Courbe de Kaplan Meier

La méthode de Kaplan-Meier permet d'estimer la probabilité de survie avec son intervalle de confiance à 95 % pour des données censurées à droite et de tracer des courbes de survie (Kaplan and Meier, 1958; Rothman, 1978). Les intervalles de temps débutent à l'instant *t* où survient un décès et se terminent juste avant le décès suivant.

### Modèle de Cox

Le modèle de Cox prend en compte l'effet de facteurs confondants explicatifs de la survie par une analyse dite multivariée (Cox, 1972; Therneau and Grambsch, 2000). L'effet d'une variable sur la survie est modélisé après ajustement sur les autres variables explicatives de décès introduites dans le modèle.

Survie globale : la survie globale a été définie comme la date d'entrée dans le protocole jusqu'à la mort de la patiente ou la date de dernières nouvelles pour les patientes toujours en vie au dernier contact.

### Résultats

L'étude de la diversité combinatoire du TCR au moment de la rechute chez des patientes atteintes d'un cancer du sein permet de prédire le risque de décès précoce (< 6 mois) et ainsi d'identifier un sous-groupe de patientes vraisemblablement réfractaires au traitement de référence et donc éligibles pour l'accès à des innovations thérapeutiques dans le cadre d'essais cliniques.

Une étude statistique univariée réalisée sur une cohorte de 66 patientes a montré qu'une diversité combinatoire de la chaîne β du TCR <20% est un marqueur d'un risque de décès précoce (Fig.1-C). Ce marqueur est significatif dès le seuil de diversité de 30% (Fig. 1 B) et est optimum à 20% (Fig.1 C). 6/6 patientes à diversité <20% sont décédées avec une médiane de survie de 5.21 mois *vs.* 37/60 décès pour les patientes à diversité 20% avec une médiane de survie de 23.2 mois.

Une analyse multivariée confirme les résultats observés en univarié lorsque la divpénie est intégrée dans un modèle de prédiction simple validé (Tableau 2). Une diversité combinatoire hTRB <20% est un facteur indépendant (p-value <0.05) des autre facteurs pronostiques tels que le taux d'hémoglobine, le taux de PNN et la localisation hépatique des métastases. Notons que la lymphopénie (LT<700/µl) n'est pas apparue comme un facteur pronostique de décès précoce dans l'ensemble de la cohorte étudiée (P=0.35).

**Tableau 2 analyse multivariée de la diversité combinatoire TRB seuil 20% (valeur qualitative) en fonction de la survie globale**

| **FACTEURS** | **groupes** | **Taux de survie à 9 mois** | **HR** | **SD** | **P** | **IC inf 95%** | **IC sup 95%** |
|---|---|---|---|---|---|---|---|
| **hémoglobine** | **<11,5** | **44%** | **3,285** | **0,369** | **1,3.10⁻³** | **1,592** | **6,776** |
| | **= 11,5** | **78%** | | | | | |
| **PNN** | **<= 7,5** | **70%** | **1,821** | **0,567** | **0,290** | **0,599** | **5,531** |
| | **> 7,5** | **57%** | | | | | |
| **Site tumoral méta hépatiques** | **Non** | **[82%;88%]** | **0,338** | **0,450** | **0,016** | **0,140** | **0,817** |
| | **Oui** | **64%** | | | | | |
| **DlVPENIE.TRB.VJ** | **<20%** | **17%** | **4,743** | **0,598** | **0,009** | **1,470** | **15,301** |
| | **>= 20%** | **78%** | | | | | |

Il est communément admis que la mesure du nombre de PNN est un des marqueurs de risque des patients atteints de cancers ou autres pathologies infectieuses.

La lymphopénie CD4+ décrite comme un marqueur de décès précoce (Borg et al., 2004) indique que le système immunitaire, et plus particulièrement les lymphocytes T, peuvent présenter un intérêt dans le pronostic de décès précoce. Or, de manière tout à fait intéressante, l'analyse statistique multivariée avec comme référentiel une évaluation du taux de survie à 9 mois indique que la divpénie TRB <20% est un facteur pronostique plus puissant que la mesure des PNN et de la présence de métastases hépatiques. Par ailleurs, les observations des inventeurs ont montré que la mesure de la divpénie TRB est un facteur indépendant de la mesure de la lymphopénie CD4+ (p-value <0.05) (tableaux 3 & 4).

**Tableau 3 : analyse multivariée de la diversité combinatoire TRB (seuil 30%) versus numération CD4 (seuil 450 cellules/µL) (valeur qualitative) en fonction de la survie globale.**

| **FACTEUR** | **groupes** | **Taux de survie à 14 mois** | **HR** | **SD** | **p** | **IC inf 95%** | **IC sup 95%** |
|---|---|---|---|---|---|---|---|
| **CD4+** | **<= 0,450** | **28%** | **2,503** | **0,461** | **4,7.10⁻²** | **1,014** | **6,183** |
| | **>0,450** | **71%** | | | | | |
| **DIVPENIE_TRB_VJ** | **<30%** | **36%** | **2,232** | **0,431** | **6,3.10-2** | **0,959** | **5,198** |
| | **>= 30%** | **61%** | | | | | |

**Tableau 4 analyse multivariée de la diversité combinatoire TRB (seuil 20%) versus numération CD4 (seuil 450 cellules/µL) (valeur qualitative) en fonction de la survie globale.**

| **FACTEUR** | **groupes** | **Taux de survie à 9 mois** | **HR** | **SD** | **p** | **IC inf 95%** | **IC sup 95%** |
|---|---|---|---|---|---|---|---|
| **CD4+** | **<= 0,450** | **45%** | **2,489** | **0,461** | **0,048** | **1,008** | **6,148** |
| | **>0,450** | **[83%;88%]** | | | | | |
| **DIVPENIE_TRB VJ** | **<20%** | **17%** | **5,188** | **0,597** | **0,006** | **1,611** | **16,709** |
| | **>=20%** | **78%** | | | | | |

### Conclusion

Le kit ImmunTraCkeR P permet de prédire les risques de décès précoce chez les patientes atteintes de cancer du sein en situation métastatique. Ce marqueur est indépendant de la lymphopénie CD4+ (<450 CD4+/µl) décrite par Borg C & al. (2004).

### Exemple 2: Etude de la corrélation entre un risque de décès précoce accru et une faible diversité combinatoire B, chez des patientes atteintes de cancer du sein métastatique

### Matériels et méthodes

La diversité combinatoire des lymphocytes B a été étudiée en utilisant le test hIgH®.

Une PCR Multiplexe IgH® est réalisée en utilisant un oligonucléotide *"upstream"* spécifique de tous les membres d'une famille V donnée et un oligonucléotide *"downstream"* spécifique d'une famille J donnée. Cette technologie permet la détection simultanée de plusieurs réarrangements V-J dans la même réaction. Le test IgH® est composé de 8 puits chacun capable de détecter l'ensemble des réarrangements d'une famille V. Ce test permet de détecter 48 réarrangements hIgH V-J différents (donc l'observation de 48 réarrangements correspond à 100% des réarrangements observables). Les conditions de PCR sont décrites dans l'article de Gilles Marodon et al. 2009, *supra.*

Les autres matériels et méthodes sont identiques à ceux utilisés à l'exemple 1 ci-dessus.

### Résultats

La quantité de lymphocytes B a été étudiée auparavant (Borg & al. 2004), sans être identifiée en tant que marqueur de décès précoce chez les patientes atteintes de cancer du sein métastatique. La corrélation entre la diversité combinatoire IgH et la survenue d'un décès précoce a néanmoins été étudiée sur la même cohorte de patientes que celle décrite à l'exemple précédent (66 patientes).

Une analyse univariée de la survie globale en fonction de la divpénie IgH (tableau 5) n'a pas permis de mettre en évidence de résultat significatif (p-value 5%).

**Tableau 5 : analyse univariée de la diversité combinatoire (valeur quantitative et qualitative) en fonction de la survie globale**

| **REPERTOIRE** | **HR** | **SD** | **P-value** |
|---|---|---|---|
| **DIVERSITE IGH** | **0,988** | **0,009** | **0,180** |
| **DIVPENIE IGH < 60%** | **1,540** | **0,323** | **0,180** |
| **DIVPENIE IGH ≥60%** | | | |

La diversité combinatoire de la chaîne IgH a, malgré les résultats de l'analyse univariée, été intégrée dans un modèle de prédiction multivarié simple (tableau 6) comme cela avait été réalisé pour l'étude du TCR (exemple 1). Dans cette analyse, le seuil de diversité a été placé à 50% afin de se placer dans les conditions les plus favorables. Avec une p-value 5% (P=0.2) la diversité combinatoire IgH ne permet pas de prédire le décès précoce chez les patientes métastatiques.

**Tableau 6 : analyse multivariée de la diversité combinatoire IgH (seuil 50%) en fonction de la survie globale**

| **FACTEUR** | **groupes** | **Taux de survie à 14 mois** | **HR** | **SD** | **p** | **C inf 95%** | **IC sup 95%** |
|---|---|---|---|---|---|---|---|
| **hémoglobine_gp** | **< 11,5** | **28%** | **3,609** | **0,363** | **4.10⁻⁴** | **1,772** | **7,349** |
| | **>= 11,5** | **69%** | | | | | |
| **PNN_gp** | **<= 7,5** | **59%** | **1,182** | **0,544** | **0,760** | **0,407** | **3,431** |
| | **>7,5** | **43%** | | | | | |
| **site,tumoral Foie** | **Non** | **83%** | **0,239** | **0,482** | **0,003** | **0,093** | **0,616** |
| | **Oui** | **49%** | | | | | |
| **DIVPENIE_IGH** | **<50%** | **38%** | **1,849** | **0,478** | **0,200** | **0,725** | **4,717** |
| | **>= 50%** | **59%** | | | | | |

### Conclusion

Ces observations sur la diversité IgH sont valables sur la cohorte étudiée, dans le modèle utilisé, et avec la version de la technologie à la date de l'étude. Dans ces conditions, la diversité IgH ne permet pas de prédire la survie des patientes atteintes de cancer du sein métastatique.

### Exemple 3: Comparaison des coûts estimatifs pour un essai clinique pour tester un traitement innovant pour un cancer solide, avec ou sans sélection des patients selon leur niveau de diversité lymphocytaire

En considérant que le procédé de stratification à l'inclusion est moins cher que le traitement, si sur 100 patients testés par le présent procédé, 15 sont à risque, il sera possible de concentrer l'étude clinique sur les 15 patients plutôt que sur les 100 patients. Cette approche de stratification des patients ayant le plus besoin du traitement permet aussi d'augmenter les chances de succès d'obtenir une p-value significative vis-à-vis d'une approche globale qui au final pourrait aboutir à une p-value non significative. (Cf. tableau 7 ci-dessous).

**Tableau 7 : exemple d'hypothèses d'économie réalisée et d'amélioration de la p-value, suite à la stratification à l'inclusion des patients en fonction de la divpénie mesurée**

| **Exemple : 100 patients** | **Nombre** | **Cout** Exemple de cout moyen 1 0000E /patient | **Corrélation Avec efficacité de traitement** |
|---|---|---|---|
| Divpénie <20% | 15 (hypothèse) | 150 K€ | p< 0,005 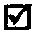 (hypothèse) |
| Divpénie >20% | 85 (hypothèse) | 850 K€ | p>0,4 (hypothèse) |
| Total | **100** | **1 M€** | p> 0,3 (hypothèse) |

### REFERENCES

Baum, P.D. and McCune, J.M. (2006) Direct measurement of T-cell receptor repertoire diversity with AmpliCot. Nat Methods, 3, 895-901.
Bogue, M., Gilfillan, S., Benoist, C. and Mathis, D. (1992) Regulation of N-region diversity in antigen receptors through thymocyte differentiation and thymus ontogeny. Proc Natl Acad Sci USA, 89, 11011-11015.
Bonarius, H.P., Baas, F., Remmerswaal, E.B., van Lier, R.A., ten Berge, I.J., Tak, P.P. and de Vries, N. (2006) Monitoring the T-cell receptor repertoire at single-clone resolution. PLoS ONE, 1, e55.
Borg, C., Ray-Coquard, I., Philip, I., Clapisson, G., Bendriss-Vermare, N., Menetrier-Caux, C., Sebban, C., Biron, P. and Blay, J.Y. (2004) CD4 lymphopenia as a risk factor for febrile neutropenia and early death after cytotoxic chemotherapy in adult patients with cancer. Cancer, 101, 2675-2680.
Chaudhuri, J., Tian, M., Khuong, C., Chua, K., Pinaud, E. and Alt, F.W. (2003) Transcription-targeted DNA deamination by the AID antibody diversification enzyme. Nature, 422, 726-730.
Cochet, M., Pannetier, C., Regnault, A., Darche, S., Leclerc, C. and Kourilsky, P. (1992) Molecular detection and in vivo analysis of the specific T cell response to a protein antigen. Eur J Immunol, 22, 2639-2647.
Cox, D.R. (1972) Regression model and life tables. J Roy Stat Soc, 34, 187-220.
Davis, M.M. and Bjorkman, P.J. (1988) T-cell antigen receptor genes and T-cell recognition. Nature, 334, 395-402.
Douek, D.C., McFarland, R.D., Keiser, P.H., Gage, E.A., Massey, J.M., Haynes, B.F., Polis, M.A., Haase, A.T., Feinberg, M.B., Sullivan, J.L., Jamieson, B.D., Zack, J.A., Picker, L.J. and Koup, R.A. (1998) Changes in thymic function with age and during the treatment of HIV infection. Nature, 396, 690-695.
Fugmann, S.D., Lee, A.I., Shockett, P.E., Villey, I.J. and Schatz, D.G. (2000) The RAG proteins and V(D)J recombination: complexes, ends, and transposition. Annu Rev Immunol, 18, 495-527.
Fuschiotti, P., Pasqual, N., Hierle, V., Borel, E., London, J., Marche, P.N. and Jouvin-Marche, E. (2007) Analysis of the TCR alpha-chain rearrangement profile in human T lymphocytes. Mol Immunol, 44, 3380-3388.
Hamblin, T.J., Davis, Z., Gardiner, A., Oscier, D.G. and Stevenson, F.K. (1999) Unmutated Ig V(H) genes are associated with a more aggressive form of chronic lymphocytic leukemia. Blood, 94, 1848-1854.
Kaplan, E.L. and Meier, P. (1958) Non parametric estimation from incomplete observations. J Am Stat Assoc, 53, 457-481.
Kotani, A., Okazaki, I.M., Muramatsu, M., Kinoshita, K., Begum, N.A., Nakajima, T., Saito, H. and Honjo, T. (2005) A target selection of somatic hypermutations is regulated similarly between T and B cells upon activation-induced cytidine deaminase expression. Proc Natl Acad Sci U S A, 102, 4506-4511.
Marodon, G., Desjardins, D., Mercey, L., Baillou, C., Parent, P., Manuel, M., Caux, C., Bellier, B., Pasqual, N. and Klatzmann, D. (2009) High diversity of the immune repertoire in humanized NOD.SCID.gamma c-/- mice. Eur J Immunol, 39, 2136-2145.
Pannetier, C., Even, J. and Kourilsky, P. (1995) T-cell repertoire diversity and clonal expansions in normal and clinical samples. Immunol Today, 16, 176-181.
Pasqual, N., Gallagher, M., Aude-Garcia, C., Loiodice, M., Thuderoz, F., Demongeot, J., Ceredig, R., Marche, P.N. and Jouvin-Marche, E. (2002) Quantitative and qualitative changes in V-J alpha rearrangements during mouse thymocytes differentiation: implication for a limited T cell receptor alpha chain repertoire. J Exp Med, 196, 1163-1173.
Pham, T., Belzer, M., Church, J.A., Kitchen, C., Wilson, C.M., Douglas, S.D., Geng, Y., Silva, M., Mitchell, R.M. and Krogstad, P. (2003) Assessment of thymic activity in human immunodeficiency virus-negative and -positive adolescents by real-time PCR quantitation of T-cell receptor rearrangement excision circles. Clin Diagn Lab Immunol, 10, 323-328.
Ray-Coquard, I., Cropet, C., Van Glabbeke, M., Sebban, C., Le Cesne, A., Judson, I., Tredan, O., Verweij, J., Biron, P., Labidi, I., Guastalla, J.P., Bachelot, T., Perol, D., Chabaud, S., Hogendoorn, P.C., Cassier, P., Dufresne, A. and Blay, J.Y. (2009) Lymphopenia as a prognostic factor for overall survival in advanced carcinomas, sarcomas, and lymphomas. Cancer Res, 69, 5383-5391.
Rothman, K.J. (1978) Estimation of confidence limits for the cumulative probability of survival in life table analysis. J Chronic Dis, 31, 557-560.
Therneau, T.M. and Grambsch, P.M. (2000) Modeling, survival data. extending the Cox Model. Statistics for biology and health, Springer Ed. Mayo Foundation, New York.
Van den Beemd, van Dongen et al. (2000), "Flow cytometric detection of clonality in mature T-cell malignancies by use of a Vb antibody kit", ISAC Abstract.

## Revendications

1. Méthode pour établir *ex vivo* ou *in vitro* un pronostic pour un individu souffrant d'un cancer solide, comprenant les étapes suivantes :
(i) à partir d'acide nucléique provenant d'un échantillon biologique contenant des lymphocytes dudit individu, mesurer le niveau de diversité du répertoire des lymphocytes T dudit individu ;
(ii) comparer le niveau de diversité mesuré à l'étape (i) à un seuil prédéterminé ;
(iii) en déduire, dans le cas où le niveau de diversité mesuré à l'étape (i) est inférieur au seuil prédéterminé, que l'individu a un risque élevé de décès précoce.

2. Méthode selon la revendication 1, dans laquelle ledit individu est atteint d'un cancer métastatique.

3. Méthode selon la revendication 1 ou la revendication 2, dans laquelle le cancer dont souffre l'individu est un cancer du sein.

4. Méthode selon l'une quelconque des revendications 1 à 3, dans laquelle, à l'étape (i), l'acide nucléique utilisé est de l'ADN génomique.

5. Méthode selon l'une quelconque des revendications 1 à 4, dans laquelle, à l'étape (i), la diversité combinatoire du répertoire des lymphocytes T est mesurée.

6. Méthode selon la revendication 5, dans laquelle la mesure de la diversité combinatoire du répertoire des lymphocytes T dudit individu est effectuée par une méthode permettant d'analyser au moins 10 réarrangements V(D)J du locus TRA ou du locus TRB.

7. Méthode selon la revendication 6, dans laquelle la mesure de la diversité combinatoire du répertoire des lymphocytes T dudit individu est effectuée par une méthode permettant d'analyser au moins 20 réarrangements V(D)J du locus TRA ou du locus TRB.

8. Méthode selon la revendication 6 ou la revendication 7, dans laquelle la mesure de la diversité combinatoire du répertoire des lymphocytes T dudit individu est effectuée par une méthode permettant d'analyser au moins 70% des réarrangements V(D)J du locus TRB.

9. Méthode selon l'une quelconque des revendications 6 à 8, dans laquelle la mesure de la diversité combinatoire du répertoire des lymphocytes T est effectuée par des PCR multi-n-plexes avec n≥2 à l'aide de combinaisons d'au moins 3 amorces, chaque combinaison d'amorces comprenant au moins les amorces hTRBJ1.6 (SEQ ID No : 1), hTRBJ2.7 (SEQ ID No : 2) et une amorce hTRBV choisie dans le groupe constitué des amorces de SEQ ID Nos : 3 à 25.

10. Méthode selon la revendication 9, dans laquelle l'analyse est réalisée en effectuant au moins 23 PCR multi-2-plexes, chaque PCR multi-2-plexe étant réalisée avec un triplet d'amorces constitué des amorces hTRBJ1.6 (SEQ ID No : 1), hTRBJ2.7 (SEQ ID No : 2) et d'une amorce hTRBV choisie dans le groupe constitué des amorces de SEQ ID Nos : 3 à 25.

11. Méthode selon l'une quelconque des revendications 1 à 10, dans laquelle l'échantillon biologique est un échantillon d'un tissu choisi parmi le sang, les globules blancs (PBMC), le thymus, un ganglion, la rate, le sein, le foie, la peau, un échantillon tumoral ou un fluide biologique (épanchement pleural, ascite).

12. Méthode selon l'une quelconque des revendications 1 à 11, dans laquelle l'échantillon biologique est un échantillon de sang total ou de PBMC.

13. Méthode selon l'une quelconque des revendications 1 à 12, dans laquelle à l'étape (i), l'ADN génomique est purifié à partir de l'échantillon biologique.

14. Méthode selon l'une quelconque des revendications 2 à 13, dans laquelle le seuil utilisé à l'étape (ii) est tel que l'espérance de survie d'un patient dont le niveau de diversité mesuré à l'étape (i) est inférieur à ce seuil est au moins deux fois moindre que l'espérance de survie généralement observée pour les patients atteints du même cancer solide métastatique que celui dont il est atteint.

15. Méthode selon l'une quelconque des revendications 1 à 14, dans laquelle à l'étape (ii), le seuil est fixé à 30% de diversité, et l'interprétation à l'étape (iii) consiste à dire que si le niveau de diversité combinatoire des lymphocytes T mesuré est inférieur audit seuil, l'individu a une espérance de vie deux fois moindre que celle généralement observée pour sa pathologie.

16. Méthode selon l'une quelconque des revendications 1 à 14, dans laquelle à l'étape (ii), le seuil est fixé à 20% de diversité, et l'interprétation à l'étape (iii) consiste à dire que si le niveau de diversité combinatoire des lymphocytes T mesuré est inférieur audit seuil, l'individu a une espérance de vie de deux à cinq fois moindre que celle généralement observée pour sa pathologie.

17. Méthode selon l'une quelconque des revendications 1 à 16, dans laquelle le patient est atteint d'un cancer du sein métastatique, et dans laquelle un niveau de diversité des réarrangements V(D)J du locus TRB inférieur à 20% est indicatif d'une espérance de survie du patient inférieure à 6 mois.

18. Méthode selon l'une quelconque des revendications 1 à 17, dans laquelle le pronostic est établi sans prendre en compte la numération lymphocytaire du patient.

19. Méthode selon l'une quelconque des revendications 1 à 18, dans laquelle le pronostic est établi en associant au seuil de diversité mesuré à l'étape (i), un ou plusieurs autre(s) paramètre(s) biologique(s) ou clinique(s) sélectionnés parmi la numération lymphocytaire, le nombre de cellules CD4+, le niveau cytokines sériques, le PS (*performance status*) et le niveau d'hémoglobine.

20. Méthode pour déterminer *ex vivo* ou *in vitro* si un patient atteint d'un cancer solide doit être inclus dans un protocole de recherche clinique pour tester un nouveau médicament, comprenant les étapes suivantes :
(i) déterminer, en mettant en oeuvre la méthode selon l'une quelconque des revendications précédentes, si le patient a un risque accru de décès précoce, et
(ii) si le patient a un risque accru de décès précoce, l'inclure dans le protocole de recherche clinique.

21. Utilisation d'une méthode selon l'une quelconque des revendications 1 à 20, pour aider le clinicien à déterminer si un patient atteint d'un cancer solide doit bénéficier d'un suivi hospitalier particulier et/ou d'une antibiothérapie préventive et/ou d'une immunostimulation et/ou d'une vaccination et/ou d'une chimiothérapie et/ou d'un changement de posologie ou de fréquence d'administration d'une chimiothérapie, et/ou de compléments alimentaires visant à reconstituer ses défenses immunitaires.


SEQUENCE LISTING

<110>  IMMUNID

<120>  UTILISATION DE LA DIVERSITE COMBINATOIRE DU REPERTOIRE DES
       LYMPHOCYTES T COMME MARQUEUR PRONOSTIC D'UN CANCER

<130>  VMAahF2110/2

<160>  25

<170>  PatentIn version 3.3

<210>  1
<211>  25
<212>  DNA
<213>  Artificial

<220>
<223>  amorce

<400>  1
cttggtgcat ggctatgtaa tcctg                                          25


<210>  2
<211>  22
<212>  DNA
<213>  Artificial

<220>
<223>  amorce

<400>  2
ctcgccctct gctcagcttt cc                                             22


<210>  3
<211>  26
<212>  DNA
<213>  Artificial

<220>
<223>  amorce

<400>  3
cacacagatg ggacaggaag tgatct                                         26


<210>  4
<211>  23
<212>  DNA
<213>  Artificial

<220>
<223>  amorce

<400>  4
gcttctcacc tgaatgcccc aac                                            23


<210>  5
<211>  25
<212>  DNA
<213>  Artificial

<220>
<223>  amorce

<400>  5
ctgatcaaaa cgagaggaca gcaag                                          25


<210>  6
<211>  25
<212>  DNA
<213>  Artificial

<220>
<223>  amorce

<400>  6
ctgatcaaaa cgagaggaca gcacg                                          25


<210>  7
<211>  23
<212>  DNA
<213>  Artificial

<220>
<223>  amorce

<400>  7
gatcacccag gcaccaacat ctc                                            23


<210>  8
<211>  25
<212>  DNA
<213>  Artificial

<220>
<223>  amorce

<400>  8
cagatcacac aggagctgga gtctc                                          25


<210>  9
<211>  27
<212>  DNA
<213>  Artificial

<220>
<223>  amorce

<400>  9
cacagatcac gcagatactg gagtctc                                        27


<210>  10
<211>  23
<212>  DNA
<213>  Artificial

<220>
<223>  amorce

<400>  10
cgcacaacag ttccctgact tgc                                            23


<210>  11
<211>  27
<212>  DNA

<213>  Artificial

<220>
<223>  amorce

<400>  11
ttcacagttg cctaaggatc gattttc                                          27


<210>  12
<211>  27
<212>  DNA
<213>  Artificial

<220>
<223>  amorce

<400>  12
ttctctggta cagacagacc tttgtgc                                          27


<210>  13
<211>  27
<212>  DNA
<213>  Artificial

<220>
<223>  amorce

<400>  13
ttttctggta cagagatacc ttcgtgc                                          27


<210>  14
<211>  25
<212>  DNA
<213>  Artificial

<220>
<223>  amorce

<400>  14
gttgctgaag tgtcaaactc tcccg                                            25


<210>  15
<211>  24
<212>  DNA
<213>  Artificial

<220>
<223>  amorce

<400>  15
tccccagcca cagcgtaata gaga                                             24


<210>  16
<211>  24
<212>  DNA
<213>  Artificial

<220>
<223>  amorce

<400>  16
ccccaaagct gctgttccac tact                                             24

<210>  17
<211>  22
<212>  DNA
<213>  Artificial

<220>
<223>  amorce

<400>  17
ctcctggtga agaagtcgcc ca                                                22


<210>  18
<211>  22
<212>  DNA
<213>  Artificial

<220>
<223>  amorce

<400>  18
tagtgcgagg agattcggca gc                                                22


<210>  19
<211>  23
<212>  DNA
<213>  Artificial

<220>
<223>  amorce

<400>  19
ctgggagcaa gtgagtcctg ggt                                               23


<210>  20
<211>  24
<212>  DNA
<213>  Artificial

<220>
<223>  amorce

<400>  20
tcatcaacca tgcaagcctg acct                                              24


<210>  21
<211>  24
<212>  DNA
<213>  Artificial

<220>
<223>  amorce

<400>  21
agtgtctctc gacaggcaca ggct                                              24


<210>  22
<211>  23
<212>  DNA
<213>  Artificial

<220>
<223>  amorce

<400> 22
cctctttgtt gggtttgtgc ctg                                              23


<210> 23
<211> 22
<212> DNA
<213> Artificial

<220>
<223> amorce

<400> 23
gtccccttcc tttacaggcc cc                                               22


<210> 24
<211> 21
<212> DNA
<213> Artificial

<220>
<223> amorce

<400> 24
ccatcagccg cccaaaccta a                                                21


<210> 25
<211> 26
<212> DNA
<213> Artificial

<220>
<223> amorce

<400> 25
tgctcttcta ctccgttggt attggc                                           26